# EUROPEAN PATENT APPLICATION

(11) **EP 4 715 382 A1**
(43) Date of publication of application: **25.03.2026**
(21) Application number: 24811350.8
(22) Date of filing: 17.05.2024
(51) Int. Cl.: G01N 33/543

(54) **AUTOMATIC ANTIBODY COUPLING AND DEVICE FOR CONFIRMING SAME**

(30) Priority: 19.05.2023 KR 20230064781
(71) Applicant: Ezdiatech Inc., Chungcheongnam-do 31116 (KR)
(72) Inventor: JUNG, Yong-Gyun, Seoul 05792 (KR); CHOI, Heelak, Ansan-si Gyeonggi-do 15345 (KR)
(74) Representative: Prüfer & Partner mbB Patentanwälte · Rechtsanwälte
(86) International application number: PCT/KR2024/006722
(87) International publication number: WO 2024/242411

(57) **Abstract**

The present invention provides an apparatus for automated antibody coupling and confirmation thereof, which can automatically perform the production of magnetic particles used for multi-diagnosis, and can perform quality control of magnetic particles and diagnosis using magnetic particles simultaneously or sequentially using the same system.

## Description

### [Technical Field]

The present invention relates to an apparatus for automated antibody coupling and confirmation thereof, and more specifically, to an apparatus for automated antibody coupling and confirmation thereof, which can automatically perform the manufacture of magnetic particles used for multi-diagnosis, and can simultaneously or sequentially perform quality control of magnetic particles and diagnosis using magnetic particles using the same system.

### [Background Art]

*In vitro* diagnosis (IVD) is a technology that allows diagnosis of health conditions by analyzing objects such as blood, urine, and cells collected from the human body, and includes immuno-diagnosis, self-blood glucose measurement, and molecular diagnosis technologies. Among these, the immune-diagnosis technology is based on measuring the presence or absence of specific proteins that cause diseases through an antigen-antibody reaction, and can be used to diagnose and track a wide variety of diseases. However, the immune-diagnosis technology has a disadvantage in that diagnosis is only possible when the target protein is present in a concentration higher than the detection limit, and thus a variety of techniques have been developed to amplify low-concentration protein generation signals.

In particular, recently, with the development of synthetic chemistry and life sciences, the target materials to be analyzed are diversified in the fields of new drug development and diagnosis, various methods using magnetic particles have been reported as one of the detection methods that guarantees high-sensitivity signal generation for detecting trace amounts of target material (Modern magnetic immunoassay: Biophysical and biochemical aspects (2017) Regul. Mech. Biosyst., 9(1), 47-55).

Conventional immuno-diagnosis using magnetic particles has used magnetic particles of nanometer size, and has utilized those whose surfaces have been modified with silica. However, magnetic particles with surfaces modified by silica are difficult to effectively extract or release from the inside of the well where the immune response is performed. In other words, these magnetic particles, which are small in size, are often floating in solution, thereby making them difficult to separate, and thus, they are often used for qualitative analysis rather than quantitative analysis. In addition, there is a problem that conventional analysis methods can only examine one magnetic particle at a time, thereby making the examination time-consuming and complicated.

Meanwhile, in the case of the magnetic particles as described above, they are used with various antibodies, proteins or nucleic acids attached to their surfaces. These antibodies, proteins or nucleic acids can selectively bind to the organism to be detected, thereby performing immuno-diagnosis, etc.

However, in the case of existing immuno-diagnosis technology, diagnosis and interpretation are automated, but in the case of manufacturing and quality inspection of magnetic particles, if small quantities are manufactured, researchers manufacture them themselves, and even if large quantities are manufactured, workers or researchers are still manually controlling each step.

In the case of such manual control, the yield of desired magnetic particles may vary depending on the skill level of the worker or researcher, and also, since highly skilled workers and researchers cannot be deployed to each application, there is a disadvantage that the desired magnetic particles cannot be supplied at the desired time.

Recently, as the use of magnetic particles increases in various fields, there is a need for various types of magnetic particles. However, if such manual manufacturing system is used, production dictated by demand cannot be performed, and also in the case of inspections using only small amount of magnetic particles, it may be difficult to secure magnetic particles at the desired time.

Therefore, there is a need for a new type of system to manufacture magnetic particles to solve these problems.

### [Disclosure]

### [Technical Problem]

In order to solve the problems described above, the present invention is intended to provide an apparatus for automated antibody coupling and confirmation thereof, which can automatically perform the production of magnetic particles used for multi-diagnosis, and can simultaneously or sequentially perform quality control of the magnetic particles and diagnosis using the magnetic particles by using the same system.

### [Technical Solution]

In order to solve the problems described above, the present invention provides an apparatus for automated antibody coupling and confirmation thereof, which comprises a cartridge including a plurality of wells; magnetic bar installed on the upper part of the wells in the cartridge and moving up and down to enter the inside of the wells; and an image acquisition unit capable of photographing the inside of one or more of the wells, wherein at least one of a cartridge for automated antibody coupling and a cartridge for confirmation after antibody coupling is selectively used as the cartridge, and at least one well in the cartridge is loaded with magnetic particles.

In one embodiment, the magnetic particle comprises a core including a magnetically responsive metal; a shell layer having a uniform thickness surrounding the core; and a capture probe introduced onto the shell layer to capture biomaterials, and the magnetic particle may be a cut piece from a glass-coated metal microwire.

In one embodiment, the apparatus for automated antibody coupling and confirmation thereof may further comprise tip cover that surrounds the lower part of the magnetic bar and moves up and down simultaneously with or separately from the magnetic bar.

In one embodiment, the cartridge for the automated antibody coupling may comprise a first well loaded with the magnetic particles; a second well loaded with EDC and NHS; a third well loaded with capture antibodies; a fourth well containing a blocking buffer; and a fifth well containing a washing buffer.

In one embodiment, the first well and the second well may further contain a 2-[N-morpholino]ethanesulfonic acid (MES) buffer.

In one embodiment, a buffer well for buffering may be further comprised between the fourth well and the fifth well, and the third well and the buffer well for buffering may contain a 4-(2-hydroxyethyl)-1-piperazineethane sulfonic acid (HEPES) buffer.

In one embodiment, the cartridge for confirmation after antibody coupling may comprise a first well loaded with magnetic particles coupled with capture antibodies; a second well loaded with second antibodies; a third well loaded with fluorescent particles; and a fourth well containing a wash buffer.

In one embodiment, the second antibody can be bound to the capture antibody through an antigen-antibody reaction, and the fluorescent particle can be bound to the second antibody.

In one embodiment, the first to third wells may further contain a blocking buffer.

In one embodiment, the cartridge may further comprise a cartridge for immuno-diagnosis.

In one embodiment, the cartridge for immuno-diagnosis may comprise a first well loaded with magnetic particles coupled with capture antibodies; a second well loaded with antigens; a third well loaded with detection antibodies; a fourth well loaded with fluorescent particles; and a fifth well containing a wash buffer.

In one embodiment, the magnetic particles coupled with the capture antibodies are a mixture of magnetic particles having two or more different lengths, and the magnetic particles having different lengths may contain different capture antibodies.

In one embodiment, the apparatus for automated antibody coupling and confirmation thereof can perform a coupling process of the magnetic particles and a confirmation process of the coupled magnetic particles as the magnetic bar moving up and down and horizontally.

### [Advantageous Effects]

The apparatus for automated antibody coupling and confirmation thereof according to the present invention can perform manufacturing and quality inspection of magnetic particles simultaneously or sequentially within the same system, and thus can perform manufacturing and quality control of magnetic particles using one system.

In addition, the apparatus for automated antibody coupling and confirmation thereof according to the present invention enables even unskilled personnel to manufacture magnetic particles in the same manner as skilled personnel, and thus enables diagnosis at a lower cost than conventional immuno-diagnosis.

In addition, the apparatus for automated antibody coupling and confirmation thereof according to the present invention can perform not only the production and quality inspection of magnetic particles, but also immuno-diagnosis within the same system, and thus enables independent performance of immuno-diagnosis even with a minimum of manpower and equipment.

### [Description of Drawings]

FIG. 1 is a schematic diagram of four important technologies used in an apparatus for automated antibody coupling and confirmation thereof according to one embodiment of the present invention.
FIG. 2 illustrates an apparatus for automated antibody coupling and confirmation thereof according to one embodiment of the present invention and a cartridge used therein.
FIG. 3 illustrates a method for manufacturing magnetic particles according to one embodiment of the present invention.
FIG. 4 shows images from a microscope of the diameter, length, etc. of the magnetic particle according to one embodiment of the present invention.
FIG. 5 illustrates a process of chemical surface treatment of the magnetic particle according to one embodiment of the present invention.
FIG. 6 illustrates an example of a length coding system for magnetic microrods performing a multi-detection function according to one embodiment of the present invention.
FIG. 7 illustrates a flow chart of automated antibody coupling according to one embodiment of the present invention.
FIG. 8 illustrates the configuration of a cartridge for automated antibody coupling according to one embodiment of the present invention.
FIG. 9 illustrates the confirmation process after antibody coupling according to one embodiment of the present invention.
FIG. 10 illustrates the configuration of the cartridge for confirmation after antibody coupling according to one embodiment of the present invention.
FIG. 11 illustrates an immuno-diagnosis process according to one embodiment of the present invention.
FIG. 12 illustrates the configuration of a cartridge for immuno-diagnosis according to one embodiment of the present invention.

### [Best Mode]

Hereinafter, preferred embodiments of the present invention will be described in detail. In describing the present invention, if it is determined that a detailed description of related known technologies may obscure the gist of the present invention, the detailed description thereof will be omitted. Throughout the specification, it is to be understood that the singular forms comprise plural referents unless the context clearly dictates otherwise, and it is to be understood that the terms such as "comprise" or "have" as used in the present specification are intended to designate the presence of stated features, numbers, steps, operations, components, parts or combinations thereof, but not to preclude the possibility of the presence or addition of one or more other features, numbers, steps, operations, components, parts, or combinations thereof. In addition, in performing the method or preparation method, each process constituting the method may occur in a different order from the specified order unless a specific order is clearly described in context. That is, each process may occur in the same order as specified, may be performed substantially simultaneously, or may be performed in the reverse order.

The technology disclosed in this specification is not limited to the embodiments described herein and may be embodied in other forms. However, the embodiments introduced herein are provided so that the content disclosed herein may be thorough and complete, and the technical spirit of the present technology may be sufficiently understood by those skilled in the art. In the drawings, in order to clearly express the components of each device, the size of the components, such as width or thickness, is shown somewhat enlarged. Overall, when describing the drawings, it was described from the observer's point of view, and when one element is referred to as being located on another element, this comprises all meanings that one element may be located directly on another element or additional elements may be interposed between them. In addition, those skilled in the art will be able to implement the spirit of the present invention in various other forms within the scope that does not depart from the technical spirit of the present invention. In addition, the same reference numerals on a plurality of drawings refer to elements that are substantially the same as each other.

In this specification, the term 'and/or' comprises a combination of a plurality of recited items or any one of a plurality of recited items. In this specification, 'A or B' may comprise 'A', 'B', or 'both A and B'.

The present invention relates to an apparatus for automated antibody coupling and confirmation thereof, comprising a cartridge including a plurality of wells; magnetic bar installed on the upper part of the wells in the cartridge and moving up and down to enter the inside of the wells; and an image acquisition unit capable of photographing the inside of one or more of the wells, wherein at least one of a cartridge for automated antibody coupling and a cartridge for confirmation after antibody coupling is selectively used as the cartridge, and at least one well in the cartridge is loaded with magnetic particles.

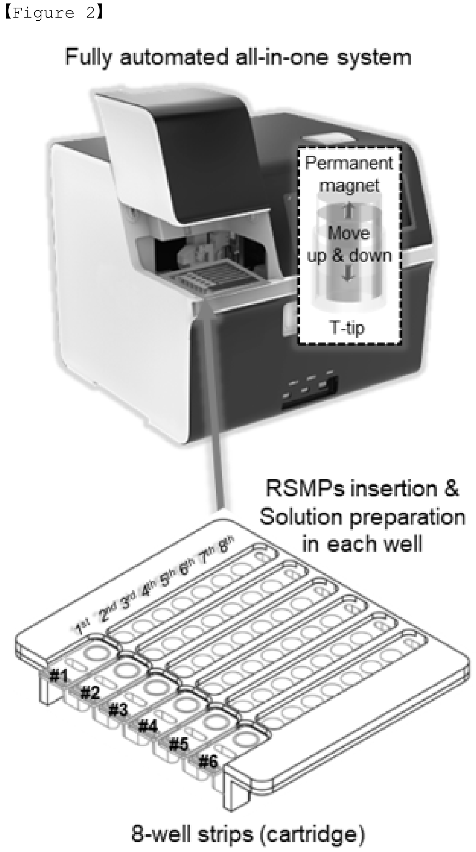

The wells refer to spaces loaded with magnetic particles, buffers, antibodies, etc., and may have a size that allows the magnetic bar described later to enter from the top. In addition, the cartridge may have a shape in which the wells described above are arranged in a single row, and the cartridge may be sealed at the top so as to be isolated from the outside until use. In addition, although the cartridge may be constructed of a single row of wells as described above, it is also possible to fabricate the cartridges so that they are connected at the sides of each cartridge, and thus the wells have a grid-like arrangement (see FIG. 2).

The magnetic particles may be loaded into one or more of the wells. In the case of the present invention, the magnetic particles may preferably be loaded into the first well.

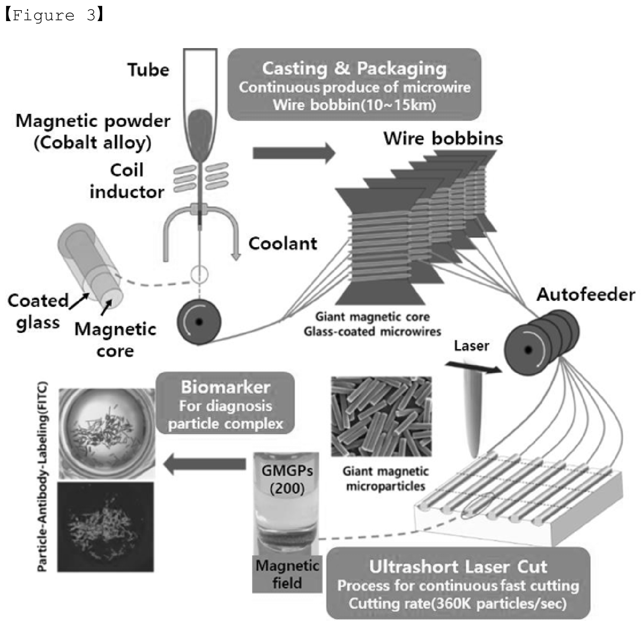

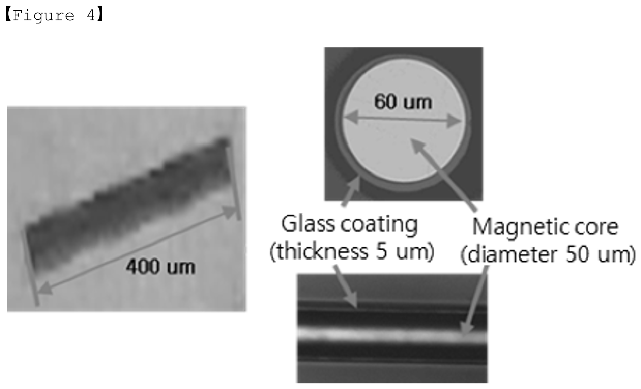

The magnetic particle comprises a core including a magnetically responsive metal; a shell layer having a uniform thickness surrounding the core; and a capture probe introduced onto the shell layer to capture biomaterials, and the magnetic particle may be a cut piece from a glass-coated metal microwire (see FIGS. 3 and 4).

The core may comprise a magnetically responsive metal, preferably a magnetic metal or a metal alloyed with a magnetic metal. The magnetic metal may be an alloy containing iron (Fe), nickel (Ni), cobalt (Co), and manganese (Mn). The magnetically responsive metal may include transition metals such as iron, nickel, cobalt, manganese, etc. as main components, and rare earth metals such as gadolinium (Gd), terbium (Tb), samarium (Sm), and other elements such as boron (B), silicon (Si), and carbon (C). Representative examples of the magnetically responsive metal may comprise an iron alloy or a cobalt alloy. A specific example of the iron alloy may be Fe70B15Si10C5, and an example of the cobalt alloy may be Co68Mn7Si10B15. In addition, the magnetic metal may be mixed or alloyed with copper (Cu), gold (Au), silver (Ag), iron (Fe), or platinum (Pt), and more preferably, may comprise a superparamagnetic metal. The core does not have magnetism directly, but it can be magnetized as a magnet is approached, and thus it can have an attractive force with the magnet and thus can be usefully used for movement and mixing using the magnet. However, in the case of the present invention, it is preferable to use a paramagnetic material rather than a ferromagnetic material. The reason for this is to reduce the phenomenon of agglomeration due to the magnetic attraction between each magnetic particle when the magnet is removed. However, if a ferromagnetic material is used, it may not be easy to observe the individual fluorescence of each particle due to this agglomeration phenomenon. Therefore, it is preferable that the core of the present invention uses paramagnetic particles. In addition, it is preferable that the magnetic particles have a size and specific gravity that prevent them from floating on water so that they can be quickly collected or separated by an external magnet such as a permanent magnet or an electromagnet.

The core may occupy 60% or more of the total volume of the magnetic particle. For example, the core may be 60 to 99%, preferably 75 to 99%, of the total volume of the magnetic particle. In addition, it is preferable that the magnetic particles have a size (e.g., diameter or length) of tens to hundreds of µm, and a specific gravity of 5 or more, for example, 5 or more and 30 or less. If the magnetic particles are nanoparticles with a size of less than 1 micrometer, they can float in water even if they have a high specific gravity (e.g., 7.876 for iron). In that case, during the bioassay process that detects biomaterials in a well using magnetic particles, it is not easy to control the magnetic particles by magnetism due to the low magnetic force of the fine particles when a magnetic field is applied, and thus it may be difficult to separate them. When the magnetic bar is moved up and down within the well to promote an immune response, fine particles are detached and attached. In that case, when the magnetic bar is moved up and down, the magnetic particles that are once attached to the magnetic bar may not fall back to the bottom of the well due to their low weight even when the magnetic field is removed, but may remain on the magnetic bar through nonspecific binding. In that case, when detecting biomaterials within the well, the reproducibility of quantitative analysis may be reduced.

Meanwhile, the magnetic particle has a core-shell structure by having a magnetically responsive metal at the center and a shell layer surrounding it. The shell layer can be made of an organic or inorganic material, and is preferably glass.

The glass may contain as a main component a compound selected from the group consisting of soda lime, borosilicate, aluminosilicate, silica, alkali silicate, Pyrex, and quartz. Preferably, the glass is for experiments requiring heat resistance, acid resistance, and water resistance, and may be borosilicate that can minimize nonspecific binding to antibodies.

The surface of the shell may contain a hydrophilic functional group.

In the case of the surface of the shell, it may be bound with the capture probe described later, including various functional groups, wherein the functional group is a hydrophilic functional group, and the hydrophilic functional group may be a silanol group or a carboxyl group, an amino group, a hydroxyl group, a thiol group, or an aldehyde group derived from the silanol group. More preferably, the functional group may be a silanol group.

In addition, the hydrophilic functional group may be directly bonded to the capture probe, but preferably may be bonded using a linker. The linker is positioned between the hydrophilic functional group and the capture probe to allow the capture probe to be easily attached to the shell layer. Preferably, a silane compound, more preferably, amino-silane, and most preferably, 3-aminopropyltrimethoxysilane (APTES) may be used.

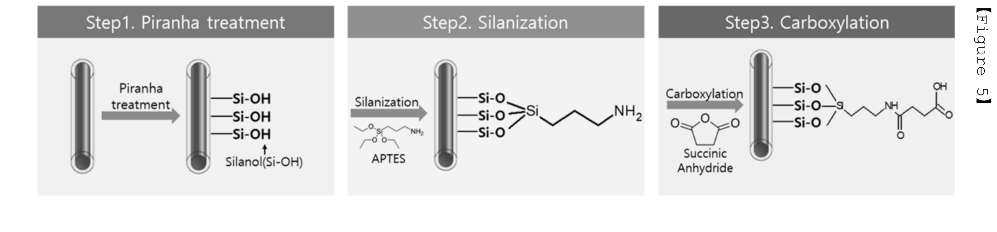

The silane compound may preferably have a structure of H2N-L-Si(OR)3. In that case, L is C1 to C12 alkylene or C6 to C20 arylene, or those formed by arbitrarily combining the alkylene and the arylene. Also, a part of the main chain of L may be substituted with 0 to 4 -NH-, and R is hydrogen or C1 to C8 alkyl (see FIG. 5).

The silane compound may preferably be an amino-silane, and as a specific example of the amino-silane, it may be at least one selected from the group consisting of 3-aminopropyltriethoxysilane, aminosilane Hydrolysate, 3-aminopropyltrimethoxysilane, 2-aminoethyl-3-aminopropyltrimethoxysilane, 2-aminoethyl-3-aminopropyltriethoxysilane, 2-aminoethyl-3-aminopropyl methyldimethoxysilane, 3-aminopropylmethyldiethoxysilane, 3-aminopropyltrimethoxysilane, N-2-aminoethyl-3-aminopropyltriethoxysilane, triethoxysilylpropyldiethylenetriamine, bis(trimethoxysilypropyl)amine, and bis(triethoxysilypropyl)amine. Preferably, considering economic efficiency, 3-aminopropyltrimethoxysilane (APTES) may be used. In one embodiment, the amine terminal may be substituted with a carboxyl group so as to be easily coupled to the capture probe.

In addition, the shell layer may substantially completely surround the magnetic particle, but when necessary or during the manufacturing process, the surface of the fine particle may not be completely covered by the shell layer and some areas of the surface of the magnetic particles may be exposed.

In order to capture the biomaterials in the sample solution, the magnetic particle according to one embodiment of the present invention may have a capture probe introduced on the shell layer. The capture probe specifically binds to the antibody described below and serves to capture the antibody to the magnetic particle. The capture probe can be fixed by adsorption to the surface of the magnetic particle or by chemical linkage, and preferably can be linked to a hydrophilic functional group existing on the surface of the shell layer. For example, biotin can be introduced to the surface of the capture probe, and avidin, neutravidin, or streptavidin, which can bind to biotin, can be introduced to the magnetic particle to attach the capture probe to the magnetic particle. In that case, the capture probe can be connected to the magnetic particle by using the hydrophilic functional group on the surface of the magnetic particle.

In that case, the hydrophilic functional group may be directly bonded to the capture probe, but may preferably be bonded using a linker. The linker may be a silane compound.

The core-shell structure may be formed by applying a liquid shell component to a core metal or by filling a hollow mold with a core metal component.

The shell layer may be solidified from a liquid coating solution of an organic or inorganic substance. More specifically, the shell layer can be formed by making a liquid coating solution through a method of melting an organic or inorganic shell component at high temperature or making it flowable or a method of dissolving it in a solvent, and then applying it to the core metal. The organic material may be mainly a polymer, and the inorganic material may be a metal or ceramic, particularly glass. For example, to form the shell layer, a coating solution obtained by dissolving plastic in a solvent or melting glass can be applied to the magnetic particle by methods such as dip coating or spray coating.

The cartridge for the automated antibody coupling may be loaded with magnetic particles having the same length. That is, in the case of the present invention, magnetic particles to which the same antibodies are bound can be manufactured in a single cartridge for automated antibody coupling, and when magnetic particles to which various antibodies are bound are required, a plurality of cartridges for the automated antibody coupling can be connected in parallel and used. Looking into this in detail, in the case of the cartridge for automated antibody coupling of the present invention, the cartridge for automated antibody coupling may be supplied in a sealed state with the magnetic particles loaded therein to prevent contamination. In that case, when loading multiple types of antibodies, the absolute amount of magnetic particles produced may be decreased, and also, the sensitivity may be decreased or only some antibodies may have high sensitivity, due to competition between each antibody. In addition, when loading multiple types of antibodies, multiple types of antibodies bind to magnetic particles having the same length, and thus multiple detections according to length may not be performed. Therefore, in the case of the present invention, the cartridge for automated antibody coupling is loaded with magnetic particles having the same length, and it is preferable that only one antibody is coupled.

The magnetic particle can be a cut piece from a glass-coated metal microwire. The magnetic particles having various lengths can be easily obtained by simply cutting the glass-coated metal microwire with a laser.

The magnetic particles may have various shapes including regular shapes such as rods, flat plates, spheres, etc., or irregular shapes. Even when the magnetic particles have a flat plate shape, the cross-section can have various shapes such as a star shape, a polygon shape, a circle shape, etc., and is not particularly limited. Preferably, the magnetic particles are preferably in the form of microrods, microdiscs, or microbeads for the convenience of manufacturing and ease of observation, and are particularly preferably in the form of microrods. If the magnetic particles have the shape of microrods, it is easy to distinguish between magnetic particles by overlapping, and when placed in a well, focusing is easy, and since the area occupied by each particle is small, a large number of fine particles can be observed on a single observation screen. On the other hand, if the magnetic particles have a shape with a complex cross-section, such as a star shape, since breakage of the corners may occur due to collisions with each other or the walls during movement inside the well, it is more advantageous to have a simple shape such as a microrod.

The length of the microrod may be 10 to 1,000 µm. If the length of the microrod is less than the above range, it is not easy to distinguish between particles of different lengths, and as the size of the particles decreases, the magnetism is decreased, which may make it difficult to mix, wash, and move using a magnet. In addition, when the above range is exceeded, the particles may overlap, making observation difficult, and it may be difficult to put the desired amount of magnetic particles into one well, making smooth diagnosis difficult. In addition, the ratio of the length to the diameter of the microrod (aspect ratio) may be 2 or more, 5 or more, or 10 or more, and the aspect ratio may be preferably 20 or less. If the aspect ratio is less than 2, it may be difficult to distinguish them from spherical particles because they are similar to each other. If the aspect ratio exceeds 20, as the diameter of the magnetic particles is decreased, the core portion that has relative magnetism becomes thinner, which may weaken the magnetism. In addition, when the aspect ratio exceeds 20, the durability may be reduced due to the thin diameter, and the magnetic particles may break during inspection.

As the cartridge, at least one of a cartridge for automated antibody coupling and a cartridge for confirmation after antibody coupling can be selectively used.

In the case of conventional antibody coupling, it is usually produced based on labor force in a laboratory. This manufacturing method based on labor force has the advantage of being easy to manufacture small quantities of antibody-coupled particles. In particular, in the case of particles used in an antibody-based diagnostic method such as the present invention, since multiple types of particles are required and at the same time, each antibody must be coupled, a manufacturing method based on labor force suitable for small-scale production is widely used.

However, in the case of such manufacturing methods based on labor force, the yield of coupled particles can vary greatly depending on the skill of the manufacturer, and there are many cases of false negatives when using particles with low yields, and thus it is very important to retain skilled personnel. In addition, even when manufactured by skilled personnel, there is a problem that due to the nature of the manufacturing method based on labor force, different amounts of reagents are often used for each manufacturing process, and thus the yield is not constant.

In the case of the present invention, by using the cartridge and the apparatus for automated antibody coupling, it is possible to provide coupled particles with a high yield compared to manufacturing methods based on labor force while maintaining a constant yield.

In addition, in the case of the existing apparatus for automated coupling, confirmation process (QC) after the coupling is usually performed using labor force or using a different type of confirmation device. However, in the case of the present invention, confirmation of the manufactured magnetic particles can be easily performed by using the same apparatus but using a cartridge for confirmation after antibody coupling, as described above.

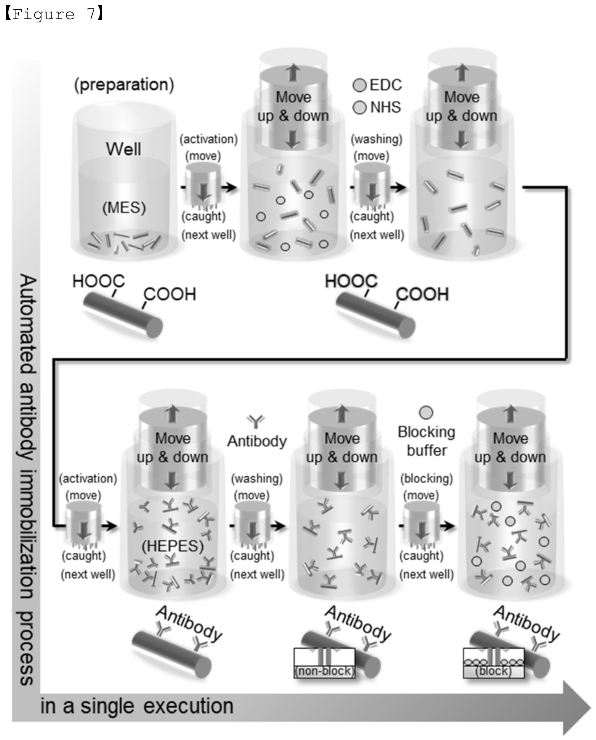

The cartridge for the automated antibody coupling may comprise a first well loaded with the magnetic particles; a second well loaded with EDC and NHS; a third well loaded with capture antibodies; a fourth well containing a blocking buffer; and a fifth well containing a washing buffer (see FIG. 7).

The magnetic particles are as described above, and thus their description will be omitted.

The magnetic particles move sequentially from the first well to the fifth well, and coupling can be performed. At that time, the movement of the magnetic particles and mixing inside the well are performed using a magnetic bar, which will be described later.

The configuration of the cartridge for automated antibody coupling is described through a method for automated antibody coupling using the cartridge for automated antibody coupling (see FIG. 7).

First, the magnetic particles loaded in the first well can be moved to the second well. The first well may be loaded with the magnetic particles, and additionally may be injected with a 2-[N-morpholino]ethanesulfonic acid (MES) buffer. Through this, the magnetic particles of the present invention can be stored for a long period of time, and can be stored in a state where they can float within the first well without additional injection of the buffer during use.

The magnetic particles moved to the second well above can react with EDC and NHS.

In the present invention, the EDC refers to 1-ethyl-3-(3-dimethylaminopropyl)carbodiimide, which is used to generate an amide bond. Looking at this in detail, EDC can be used as a carboxyl activator for coupling primary amines.

In addition, the NHS refers to N-Hydroxysuccinimide and is used simultaneously with the EDC to bind NH2 to an antibody.

When the EDC and NHS are used, NH2 can be bound to the carboxyl group (-COOH) present in the magnetic particle, and the mechanism for this is as shown in Reaction Scheme 1 below.

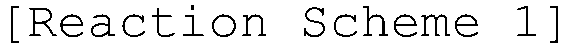

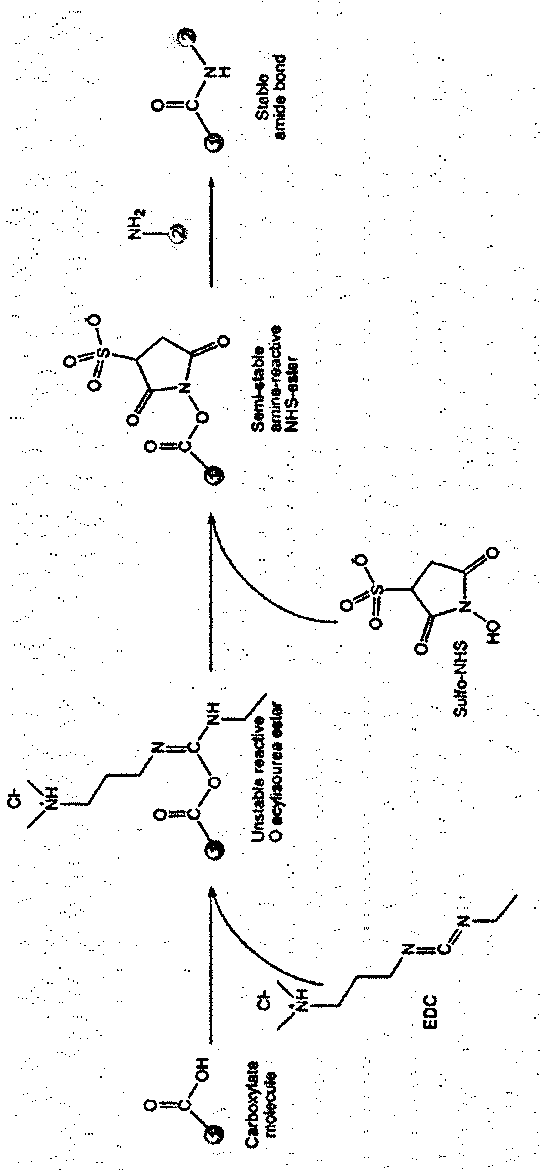

In addition, the MES buffer may be injected into the second well to increase the buffer for the reaction. In the case of EDC, the reaction can be performed at a low pH. Therefore, by using the MES buffer as described above, the pH conditions can be satisfied.

When the reaction in the second well as described above is completed, the magnetic particles can be moved to the third well. The third well is loaded with capture antibodies, and the capture antibodies can be bound to the capture probe present on the surface of the magnetic particles. In addition, at that case, if the cartridge for automated antibody coupling comprises each capture probe, the capture antibodies may also be present in an amount equal to the number of each cartridge, thereby producing magnetic particles capable of simultaneously detecting various types of antigens. The third well may further contain a 4-(2-hydroxyethyl)-1-piperazineethane sulfonic acid (HEPES) buffer. The HEPES buffer is a type of buffer for buffering and can act as a buffer for the production of carbon dioxide, and can also be used to control reaction conditions.

The magnetic particles, to which capture antibodies are bound, in the third well can be moved to the fourth well. The fourth well is loaded with a blocking buffer, and the blocking buffer can bind to a portion of the surface of the magnetic particles to which the capture antibodies are not bound. Through this, non-specific binding of antigens or fluorescent materials to the capture antibodies can be prevented, and as a result, false positives can be reduced while increasing sensitivity.

The magnetic particles bound to the blocking buffer as described above may be moved to the fifth well and washed. The fifth well is loaded with a washing buffer, and by washing in the fifth well, excess antibodies, buffers, etc. can be removed. In addition, the fifth well may be a single well, but may be composed of 2 to 5 wells, and by washing sequentially in these wells, the washing effect may be increased.

Between the fourth well and the fifth well, a buffer well for buffering may be additionally comprised. In the case of the fourth well, combination with the blocking buffer as described above can be performed, and in this process, the pH or the concentration of carbon dioxide may be changed. Therefore, in order to reduce such changes, it is desirable to add a buffer well for buffering, in which a buffer for buffering is loaded, between the fourth well and the fifth well. The buffer for buffering used at this time can be used without limitation as long as it is a buffer that can buffer changes in pH or carbon dioxide concentration, but it is preferable to use a HEPES buffer.

The magnetic particles that have been washed as described above can be stored in a blocking buffer. In addition, a storage well containing a blocking buffer may be installed at the rear end of the fifth well, i.e., the last well of the cartridge, to store the manufactured magnetic particles while loading the blocking buffer.

As described above, the antibody coupling can be performed by using the magnetic particles of the present invention starting from the first well and reaching the fifth well. However, these magnetic particles that have completed antibody coupling cannot be used as they are, and it is preferable to go through a confirmation process to confirm that the coupling has been completed accurately.

In addition, the first to fifth wells may be arranged sequentially as described above, but each well may have a different arrangement depending on the reaction conditions within the well. For example, if heating is required for the reaction within the first well and the reaction within the third well, the heating efficiency can be increased by arranging the order of the first well, third well, and second well instead of the order of the first well, second well, and third well, and the side reaction of the second well, which does not require heating, can be reduced.

In addition, unused wells may exist between each well. In general, in the case of the cartridge for automated antibody coupling and confirmation after the coupling, a standardized cartridge is used. That is, in the case of cartridges for automated antibody coupling and confirmation after the coupling, it is preferable to use cartridges having the same wells. In addition, it is desirable that the magnetic particles that have been coupled and confirmed are stored in the last well of the cartridge. In that case, if the number of required wells is less than the number of wells existing in the cartridge, it is desirable to place unused wells in the middle of the cartridge so that the magnetic particles that have completed coupling and confirmation exist in the last well. In addition, these unused wells can be used to secure the spacing between each well, which can minimize the side reaction inside the well when the internal conditions of the well are changed by heating, etc., as described above.

The configuration of the wells in these cartridges is applied not only to the cartridge for automated antibody coupling described above, but also to the cartridge for confirmation after antibody coupling and the cartridge for immuno-diagnosis described below.

As reviewed above, in the case of the existing confirmation (QC) process, it is common to perform it based on labor force or use separate equipment, but in the case of the present invention, it is possible to perform the confirmation process using the same equipment by replacing the cartridge for automated antibody coupling with the cartridge for confirmation after antibody coupling.

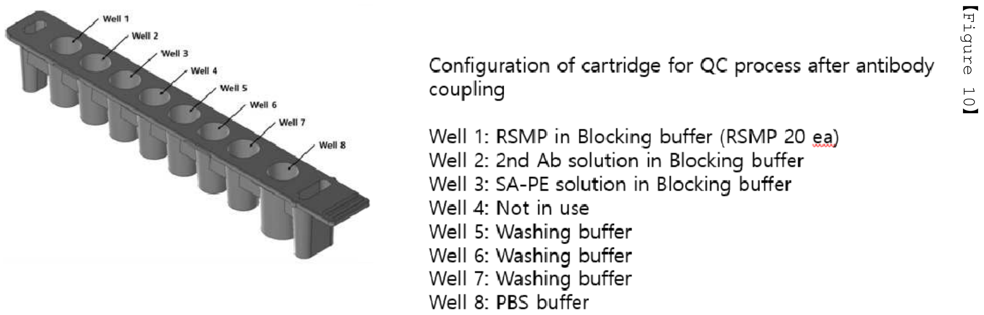

The cartridge for confirmation after the antibody coupling may include a first well loaded with magnetic particles coupled with capture antibodies; a second well loaded with second antibodies; a third well loaded with fluorescent particles; and a fourth well containing a wash buffer (see FIG. 10).

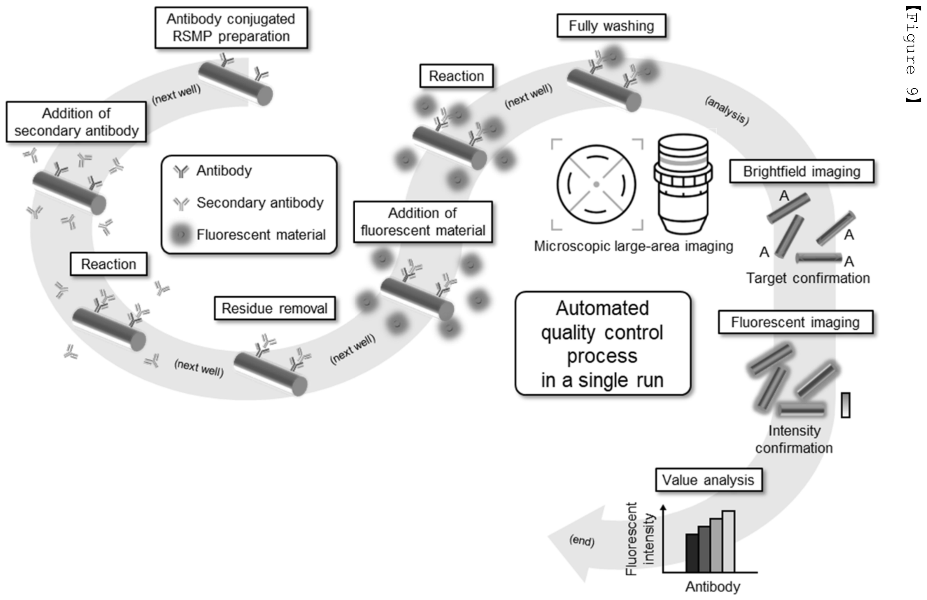

The configuration of the cartridge for confirmation after the antibody coupling will also be described according to the confirmation method after antibody coupling (see FIG. 9).

The first well may be loaded with magnetic particles coupled with capture antibodies. In that case, it is preferable that the magnetic particles coupled with the capture antibodies are magnetic particles manufactured in the cartridge for automated antibody coupling, and in addition, it is also possible to load and use magnetic particles manufactured using other methods. In addition, in the case of the cartridge for confirmation after antibody coupling, separate magnetic particles may not be loaded. In that case, it may be configured in parallel with the cartridge for automated antibody coupling, and the magnetic particles manufactured in the cartridge for automated antibody coupling may be directly transferred to the first well for use. In addition, if a plurality of cartridges for automated antibody coupling are arranged and used, the cartridge for confirmation after the antibody coupling may be positioned in the last row of cartridges for the automated antibody coupling to confirm some of the magnetic particles produced in the cartridges for the automated antibody coupling.

As described above, the magnetic particles loaded in the first well can be moved to the second well. The second well is loaded with second antibodies, and the second antibodies can be bound through an antigen-antibody reaction with the capture antibodies. That is, in the confirmation cartridge after the antibody coupling, the capture antibodies can act as antigens for the second antibodies. By using the second antibodies as described above, it is possible to confirm whether the capture antibodies are normally bound, and also since the capture antibodies do not bind to fluorescent particles, which will be described later, fluorescence detection can be performed using the second antibodies.

As described above, the magnetic particles to which the second antibodies are bound can be moved to the third well. The third well is loaded with fluorescent particles, and the fluorescent particles can bind to the second antibodies. That is, it is possible to confirm that the capture antibodies are accurately bound by binding the second antibodies and the fluorescent particles, and it is also possible to confirm the binding density of the capture antibodies by measuring the intensity of fluorescence generated by the fluorescent particles.

The fluorescent particles can be linked to the second antibodies to generate light by external stimuli such as ultraviolet rays, electron rays, chemical reactions, enzyme-substrate reactions, etc. Examples of the luminescent material include fluorescent molecules, quantum dots, metal nanoparticles, magnetic nanoparticles, enzymes, enzyme substrates, etc. Among these, in terms of availability and convenience of application, fluorescent molecules selected from the group consisting of fluorescein isothiocyanate (FITC), fluorescein, fluorescein amidite (FAM), phycoerythrin (PE), tetramethyl-rhodamine isothiocyanate (TRITC), Cyanine 3 (Cy3), Cyanine 5 (Cy5), Cyanine 7 (Cy7), Alexa fluorine dyes, and rhodamine can be commonly used as the luminescent material.

The first to third wells may contain a blocking buffer. The blocking buffer can play a role in preventing nonspecific binding by binding to a portion of the surface of the magnetic particle, to which the detection probe is not bound. Such nonspecific binding means that antigens, second antibodies, detection antibodies, or fluorescent particles bind to a part other than the detection probe, and in the case of such non-specific binding, there is a side effect of showing false positives or increasing the detection limit. Accordingly, by using a blocking buffer as described above, it can prevent non-specific binding to the surface of the magnetic particle to which the capture probe is not bound.

As described above, the magnetic particles to which fluorescent particles are bound can be moved to the fourth well and washed. The fourth well is a well loaded with a washing buffer for washing the magnetic particles, and may be composed of a single well, in the same way as the fifth well of the cartridge for the automated antibody coupling, but may also be composed of multiple wells. In the fourth well, second antibodies and fluorescent particles that are not bound to capture antibodies can be removed.

As described above, after the washing is completed, the magnetic particles can be confirmed by supplying fluorescence. In the case of magnetic particles to which capture antibodies are normally bound as described above, fluorescence can be generated by the fluorescent particles. In addition, when using a smaller number of types of capture antibodies than the number of types of magnetic particles, it can also be confirmed whether the desired type of capture antibodies is bound, by confirming that magnetic particles with the desired length are emitting light. In addition, by observing the intensity of fluorescence emitted by the magnetic particles, the binding density of the capture antibodies can be confirmed, and through this, whether the antibody coupling is accurate can also be confirmed.

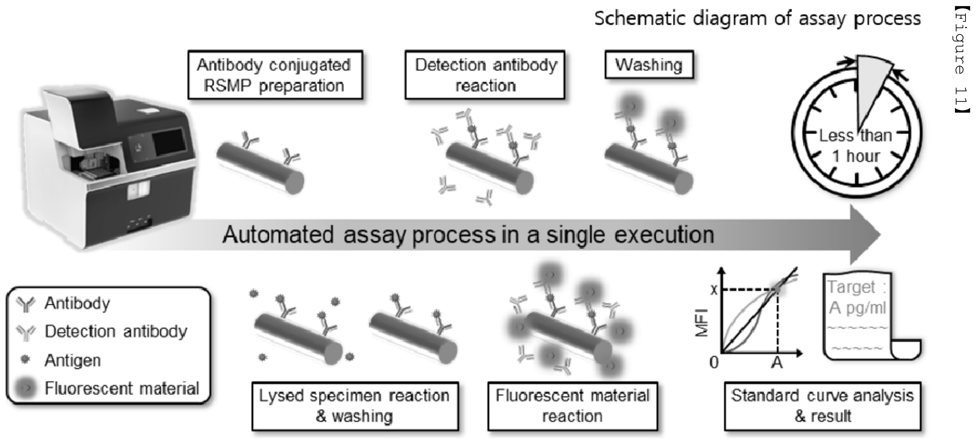

The apparatus for automated antibody coupling and confirmation thereof of the present invention can be also used for immuno-diagnosis, in addition to the automated antibody coupling and confirmation thereof as described above. In the case of the method for confirmation after antibody coupling, it is performed by almost the same process as immuno-diagnosis, which means that immuno-diagnosis can be performed by replacing some reagents and adding antigens. That is, the apparatus for automated antibody coupling and confirmation thereof of the present invention can be performed under the same process not only for antibody coupling and confirmation of coupled magnetic particles but also for immuno-diagnosis (see FIG. 11).

In addition, similarly to the cartridge for confirmation after antibody coupling, the cartridge for immuno-diagnosis can also be used by exchanging it after using the cartridge for automated antibody coupling and the cartridge for confirmation after antibody coupling, or it is also possible to connect the cartridge for automated antibody coupling and the cartridge for immuno-diagnosis in parallel so that the magnetic particles produced in the cartridge for automated antibody coupling can be moved to the first well for use.

In that case, if the number of types of magnetic particles required for the cartridge for immuno-diagnosis is n, n+m cartridges for the automated antibody coupling can be used, and it is preferable that m cartridges for confirmation after antibody coupling are used. When used in this ratio, coupling of the desired type and number of magnetic particles is possible in the cartridge for automated antibody coupling, and among these, the amount of m cartridges is confirmed in the cartridge for confirmation after antibody coupling, and the remainder can be transferred to the cartridge for immuno-diagnosis and used (see FIG. 2).

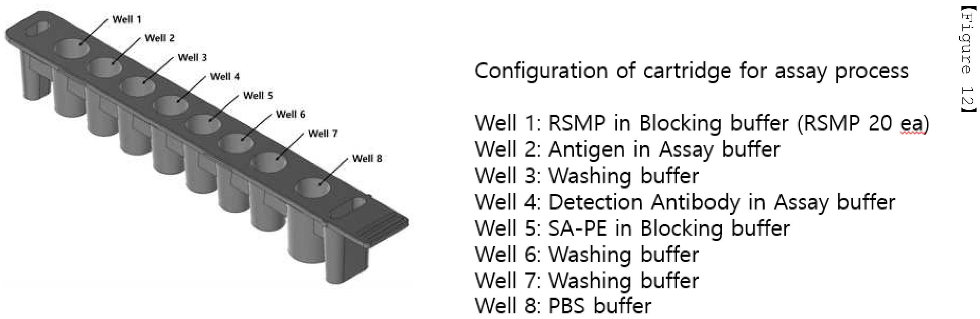

The cartridge for immuno-diagnosis may comprise a first well loaded with magnetic particles coupled with capture antibodies; a second well loaded with antigens; a third well loaded with detection antibodies; a fourth well loaded with fluorescent particles; and a fifth well containing a wash buffer (see FIG. 12).

The first well may be loaded with magnetic particles coupled with capture antibodies. In that case, the magnetic particles may be magnetic particles manufactured through another process, but preferably, they may be magnetic particles manufactured in the cartridge for automated antibody coupling and transferred thereto. As described above, in the case of the present invention, since coupling is automatically performed through the cartridge for automated antibody coupling, magnetic particles manufactured in the cartridge for automated antibody coupling can be used, and since the same process and apparatus are used, it is possible to miniaturize the apparatus and perform uniform analysis.

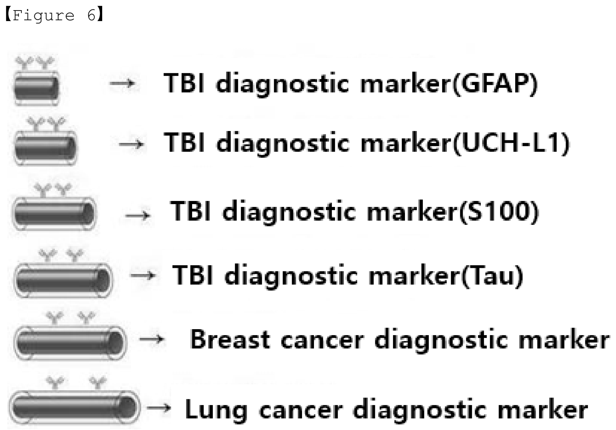

The magnetic particles to which the capture antibodies are coupled are a mixture of two or more magnetic particles having different lengths, and the magnetic particles having different lengths can include different capture antibodies (see FIG. 6). By using this, since fluorescence of different lengths is generated depending on each capture antibody, two or more biomarkers can be diagnosed simultaneously. To this end, the magnetic particles to which the capture antibodies are coupled can be a mixture of magnetic particles to which 2 to 20 types of capture antibodies are coupled, which have a difference in length of 5 to 200 µm. By using magnetic particles to which capture antibodies of various lengths are coupled, it is possible to detect 2 to 20 biomarkers simultaneously. In that case, if the difference in length between the magnetic particles to which each capture antibody is coupled is less than 5 µm, it is difficult to identify the difference in length using optical equipment, and if the difference in length exceeds 200 µm, it is easy to identify, but it is difficult to use magnetic particles to which various types of capture antibodies are coupled, making it difficult to simultaneously identify various types of biomarkers.

As an example, capture antibodies that induce an antibody-antigen reaction with GFAP can be coupled to the surface of magnetic particles having a length of 300 µm, and capture antibodies that induce an antibody-antigen reaction with UCHL-1 can be coupled to the surface of magnetic particles having a length of 400 µm. If the magnetic particles to which the two types of capture antibodies are coupled are used as a mixture, the magnetic particles to which the capture antibody is coupled generate the same fluorescence, but due to the difference in the length of the magnetic particles, it is possible to determine whether the biomarker is GFAP (fluorescence length of 300 µm) or UCHL-1 (fluorescence length of 400 µm). This means that the type of biomarker can be accurately determined simply through optical observation without using a method of genetic confirmation such as PCR.

Expanding on this, it means that if 10 different types of magnetic particles with different lengths are bound to each capture antibody and used as a mixture, it is possible to simultaneously identify 10 types of biomarkers. However, if the number of magnetic particles having different lengths exceeds 20, the quantity of each magnetic particle may be insufficient to exhibit appropriate fluorescence, and a large amount of magnetic particles must be injected to exhibit sufficient fluorescence, which may reduce efficiency.

In addition, in order to manufacture various types of magnetic particles as described above, various types of cartridges for automated antibody coupling are required as discussed above. In that case, the magnetic particles loaded into the first well of the cartridge for immuno-diagnosis, to which capture antibodies are coupled, may be derived from multiple cartridges for automated antibody coupling.

The second well is loaded with antigens. The antigens refer to detection target materials contained in the specimen, and perform an antigen-antibody reaction with antibodies coupled to the magnetic particles of the present invention.

The antigen used at this time may be a pathogen, and the pathogen may comprise bacteria, spirochaeta, rickettsia, viruses, fungi, parasites, etc. In addition, the pathogen itself can be used as a biomarker (antigen), but tissue extracts, cell lysates, proteins, DNA, RNA, etc. of the pathogen can also be used as a biomarker (antigen). In addition, when the pathogen invades, since the human body performs an antigen-antibody reaction, it is also possible to use an antibody against the pathogen as a biomarker.

In addition, in the case of the antigen, not only antigens derived from microorganisms outside the human body as described above, but also biomaterials existing inside the human body can be used as antigens. In that case, the biomaterials may be biomarkers, blood, plasma, serum, body fluid, proteins, peptides, nucleic acids, bacteria, viruses, reticulum, miRNA, exosomes, circulating tumor cells, etc. Preferably, the biomaterials may be biomarkers.

In particular, the technology disclosed in this specification can be very useful for detecting biomarkers in that it can rapidly and early diagnose diseases by detecting multiple types of biomaterials in a single test. The biomarkers may be used without limitation as long as they are used in conventional scientific or medical fields, such as measuring or evaluating biological processes, processes causing pathogenicity, pharmacological processes for treatment, etc. The biomarkers may be, for example, polypeptides, peptides, nucleic acids, proteins, or metabolites that can be detected in body fluids such as blood, saliva, urine, etc.

In addition, the specimen may be a specific sample solution derived from a living organism, and the sample solution may be tissue extracts, cell lysates, whole blood, plasma, serum, saliva, ocular fluid, cerebrospinal fluid, sweat, urine, milk, ascite fluid, synovial fluid, peritoneal fluid, etc. For rapid diagnosis, pre-treatment of the sample solution may be simplified or, in some cases, omitted.

As described above, the magnetic particles to which antigens are bound can be moved to the third well. The third well is loaded with detection antibodies, and the detection antibodies can bind to the antigens. In that case, the capture antibodies and the detection antibodies can bind to different parts of the antigens, and the bonding at each position as described above can prevent a decrease in efficiency during analysis.

The magnetic particles bound to the detection antibodies in the third well can be moved to the fourth well. The fourth well is a well loaded with fluorescent particles, and the fluorescent particles can be bound to the detection antibodies. That is, in the case of the present invention, if the antigens are present, since the detection antibodies and fluorescent particles are sequentially bound to emit fluorescence, the type of antigens can be accurately identified. In addition, since the fluorescent particles are bound only to the detection antibodies, and adsorption to different surfaces of the magnetic particles is prevented by the blocking buffer, low concentrations of antigens can be also detected.

As described above, when the binding of fluorescent particles is completed, they can be washed in the fifth well. The fifth well is loaded with a washing buffer, and unbound antibodies and fluorescent particles can be removed. In that case, the fifth well may be composed of a single well, but may be also composed of two to five wells, which are washed sequentially.

The detection in the fifth well can be performed by an immuno-diagnosis method. In particular, the detection of the luminescent signal can be performed by, for example, an ELISA method or a fluorometric method.

More specifically, the ELISA method binds the detection target materials in the sample to the capture antibodies fixed to large magnetic particles, and then processes the detection antibodies. These detection antibodies are bound to the opposite side of the detection target materials to which the capture antibodies bind. The detection antibodies are conjugated with an enzyme called Horse Radish Peroxidase (HRP), and thus the TMB substrates are decomposed by hydrogen peroxide to produce color. The degree of this color is measured as the OD value. That is, this method uses the principle that as the detection target materials in the sample are increased, the capture antibodies and detection antibodies are quantitatively bound in proportion, and as a result, the HRPs conjugated to the detection antibodies are increased, so the enzymatic reaction is proceeded relatively quickly, and thus the color of the TMB substrates is changed quickly. By drawing a standard curve using the OD values obtained by randomly processing the detection target materials at various concentrations in advance, and then comparing the OD values obtained after processing the actual sample with the standard curve, it is possible to measure the amount of detection target materials in the sample. In that case, the calculation of all OD values and concentrations can be performed by an automatic analysis program installed in the analytical instrument.

In the case of the method of measuring fluorescence, the principle of a sandwich assay using capture antibodies and detection antibodies is the same as the ELISA method, but biotin material is conjugated to the detection antibodies instead of HRP enzyme, and then fluorescent materials conjugated to streptavidin are processed to measure the fluorescence value. First, antigens or detection target materials are reacted with capture antibodies to attach them, and then the detection antibodies to which biotin material is conjugated are processed to attach the detection antibodies to the antigens or detection target materials on the other side than the place where the capture antibodies are attached to the antigens or detection target materials. Thereafter, the fluorescent material e-flour conjugated with streptavidin is processed and each fluorescent signal from the magnetic particles is measured. Based on the principle that the higher the concentration of diagnostic markers in the specimen, the stronger the fluorescent signal from the magnetic particles, the positions of the magnetic particles in the reaction well are confirmed by a bright field microscope, and the fluorescent signal corresponding to that position is measured in pixel units, and the fluorescent signals from about 10 magnetic particles are automatically image analyzed. The target substance to be detected is reacted at different concentrations, and the fluorescence value from the test detection is applied to the standard curve obtained by drawing a graph using the fluorescence value obtained by the same method to calculate the detection concentration value of the target protein to be detected in the final sample. Analysis of all images and calculation of fluorescence values are performed by an automatic image analysis program installed in the analysis instrument.

The method for immuno-diagnosis can be performed through the POCT method. In the present invention, POCT means a point-of-care test, which means performing a test directly at the place where the patient is. In other words, it means that the clinical pathology test is not performed in an independent space such as a central examination room of a hospital, but is performed by a doctor, nurse or clinical pathologist around the patient, such as in a hospital room, emergency room, operating room or intensive care unit. In the case of existing diagnosis of traumatic brain injury, on-site examination was impossible because large equipment such as CT or MRI was required, and even in the case of immuno-diagnosis, large diagnostic equipment such as automated CLIA equipment was required, and thus it was used only limitedly for on-site diagnosis. However, in the case of the present invention, since miniaturized diagnostic equipment can be used, it is possible to diagnose on-site using the POCT method.

As described above, in the case of the present invention, at least one of the cartridge for automated antibody coupling and the cartridge for confirmation after antibody coupling is used selectively or sequentially, and thus antibody coupling and confirmation after the coupling can be performed in a single process.

The magnetic bar is installed at the upper part of the well of the cartridge, and can be installed to move up and down so as to enter the inside of the well. As described above, the magnetic particles of the present invention can be manufactured to have paramagnetism. Accordingly, if the magnetic bar enters the well, the magnetic particles are attached to the magnetic bar, and if the magnetism of the magnetic bar is disappeared, the magnetic particles can be detached from the magnetic bar and thus floated inside the well. By applying this, if the magnetism of the magnetic bar is intermittently applied, the magnetic particles can be mixed inside the well, and if the magnetism of the magnetic bar is removed and then the magnetic bar is moved up and down, the fluid inside the well can be also physically mixed. In addition, it is also possible to selectively move the magnetic particles inside the well to the next well by applying the magnetism of the magnetic bar inside the well having the magnetic particles and then moving it to an adjacent well.

That is, the coupling process of the magnetic particles and the confirmation process of the coupled magnetic particles can be performed by moving the magnetic bar up and down and horizontally.

Looking at the structure of the magnetic bar in detail, it is preferable that the magnetic bar has a diameter that can enter the cartridge, and a shape that corresponds to the inner surface of the well formed in the cartridge. That is, if the well is formed in a cylindrical shape, the magnetic bar may be manufactured in a cylindrical shape with a smaller diameter than the inner surface of the well, and if the well is formed in a square column shape, the magnetic bar may have a square column shape with a small cross-sectional area. However, even if the cross-section of the well is not circular, the magnetic bar may have a cylindrical shape that can enter the well.

In addition, the magnetic bar may be a paramagnetic material that enters the well on one side and has a magnetic body coupled to the other side. As described above, the magnetic bar can also be used to mix the inside of the well. At this time, it is preferable that the magnetic bar is used in a state where the magnetization is removed. To this end, the magnetic force of the magnetic bar can be applied or removed by coupling and detaching a magnetic body to the other side of the magnetic bar, rather than making the magnetic bar itself magnetic. In that case, the magnetic body can be a permanent magnet or an electromagnet, and an electromagnet can be preferably used.

A tip cover may be further comprised, which wraps around the lower part of the magnetic bar and moves up and down simultaneously or separately from the magnetic bar. In the case of the tip cover, it can move simultaneously with the magnetic bar, and also can move separately from the magnetic bar. By using such a tip cover, the magnetic bar can be prevented from direct contact with the inside of the well, and accordingly, even when replacing the cartridge, it is possible to continue the experiment by replacing only the tip cover.

An image acquisition unit capable of photographing the inside of one or more of the wells may be further comprised. As described above, magnetic particles combined with fluorescent particles are present at the end of the cartridge for confirmation after antibody coupling, and confirmation (QC) of magnetic particles can be performed using fluorescence images in the image acquisition unit. In addition, if the cartridge includes a cartridge for immuno-diagnosis as described above, a fluorescence image can be acquired using the image acquisition unit as described above, and immuno-diagnosis can be performed based on this.

The image acquisition unit may comprise a light source, an image sensor (CCD or CMOS), and a lens, in the same way as the existing image acquisition unit. However, if the existing image acquisition unit is installed as it is, it requires a lot of space. Accordingly, in the case of the present invention, the components of the light source, image sensor (CCD or CMOS), and lens can be separated, respectively, and then installed inside the apparatus for automated antibody coupling and confirmation thereof of the present invention.

Hereinafter, preferred embodiments of the present invention will be described with reference to the accompanying drawings so that those skilled in the art can easily practice them. Also, in describing the present invention, if it is determined that a detailed description of related known function or known configuration may obscure the gist of the present invention, the detailed description thereof will be omitted. In addition, certain features presented in the drawings have been enlarged or reduced or simplified for ease of explanation, and the drawings and their components are not necessarily drawn to proper scale. However, those skilled in the art will readily understand these details.

### Preparation Example

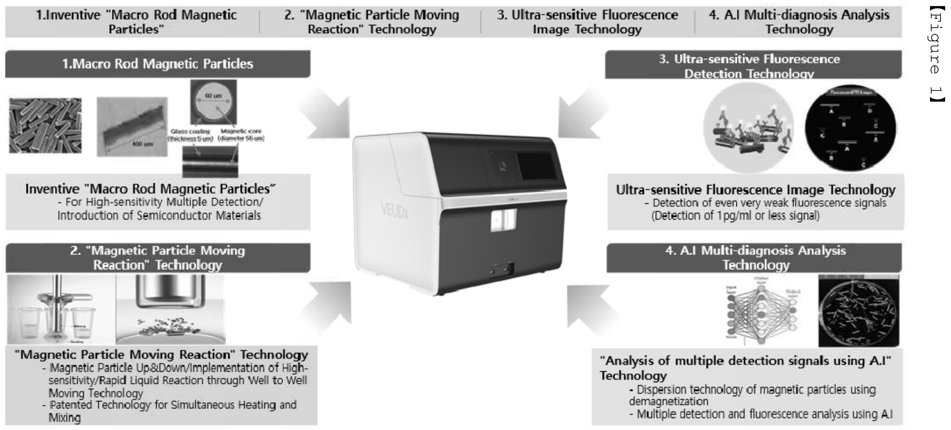

The process for automated antibody coupling and confirmation thereof was performed using the apparatus shown in FIGS. 1 and 2.

At this time, the composition of each cartridge is as follows.
Cartridge for automated antibody coupling
Well 1: magnetic particles in MES buffer
Well 2: EDC/NHS solution in MES buffer
Well 3: Capture antibody solution in HEPES buffer
Well 4: Blocking buffer
Well 5: HEPES buffer
Well 6: Washing buffer
Well 7: Washing buffer
Well 8: Blocking buffer
Cartridge for confirmation after antibody coupling
Well 1: magnetic particles in Blocking buffer
Well 2: 2nd Ab solution in Blocking buffer
Well 3: SA-PE solution in Blocking buffer
Well 4: Not in use
Well 5: Washing buffer
Well 6: Washing buffer
Well 7: Washing buffer
Well 8: PBS buffer

### (SA-PE is Phycoerythrin bound to Streptavidin)

### Example 1

The automated antibody coupling was performed using the apparatus shown in FIGS. 1 and 2 for about 2,500 magnetic particles having a length of 300 µm.

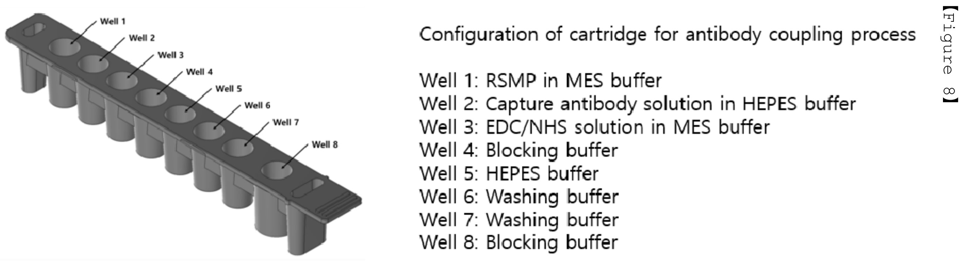

The configuration of the cartridge for antibody coupling is as shown in the Preparation Example and FIG. 8. The antibody (capture antibody) used at this time was GFAP antibody (Anti-GFAP).

Each coupling was repeated three times and then the intensity of fluorescence was measured, respectively, and also QC was performed three times for each sample using a cartridge for confirmation after antibody coupling and then the intensity of fluorescence was measured. The configuration of the QC cartridge used at this time is the same as the Preparation Example and FIG. 10.

**[Table 1]**

| | | No. 1 | No. 2 | No. 3 |
|---|---|---|---|---|
| First QC | MFI | 36777.84 | 37793.47 | 37390.64 |
| Second QC | MFI | 39412.06 | 38988.32 | 38370.90 |
| Third QC | MFI | 37406.35 | 40211.34 | 37792.90 |
| AVE | | 37865.42 | 38997.71 | 37851.48 |
| STD | | 1123.34 | 987.11 | 402.33 |
| CV(%) | | 2.97 | 2.53 | 1.06 |

As shown in Table 1, it was shown that in the case of the apparatus for automated antibody coupling and confirmation thereof of the present invention, very low dispersion (less than 3%) is shown, and thus reproducibility is very excellent. That is, it means that high accuracy can be achieved by using the apparatus for automated antibody coupling and confirmation thereof of the present invention.

### Example 2

The existing manual (labor force-based) method and the automated antibody coupling of the present invention were compared. The conditions used for each were as follows.

### Automated antibody coupling

The automated antibody coupling was performed for about 2,500 magnetic particles having a length of 300 µm using the apparatus shown in FIGS. 1 and 2.

The configuration of the cartridge for antibody coupling was as in the Preparation Example and FIG. 8. The antibody (capture antibody) used at this time was GFAP antibody (Anti-GFAP).

QC was performed three times for each sample using the cartridge for confirmation after antibody coupling (FIG. 10).

### Manual antibody coupling

The coupling was performed manually for about 2,500 magnetic particles with a length of 300 µm by researchers using the conventional method. At this time, two different researchers performed the coupling separately.

Reagents and antibodies used were the same as in automated antibody coupling.

Confirmation (QC) after antibody coupling was also performed manually as in the existing method.

**[Table 2]**

| | | Automated antibody coupling | Manual coupling #1 | Manual coupling #2 |
|---|---|---|---|---|
| First QC | MFI | 36777.84 | 29821.27 | 26468.45 |
| Second QC | MFI | 39412.06 | 33512.81 | 33548.44 |
| Third QC | MFI | 37406.35 | 30139.74 | 34155.61 |
| AVE | 37865.42 | | 31157.94 | 32057.47 |
| STD | 1123.34 | | 1670.21 | 2549.93 |
| CV(%) | 2.97 | | 5.36 | 7.95 |

As shown in Table 2, it was confirmed that in the case of the automated antibody coupling of the present invention, the dispersion is less than 3%, and thus the reproducibility is very excellent, but it was confirmed that if the coupling is performed manually, the dispersion is high, and the dispersion varies depending on the researcher, and thus the skill of the researcher is very important.

In addition, when performing the coupling and QC using the apparatus for automated antibody coupling and confirmation thereof of the present invention, the required time was measured to be 1 hour and 46 minutes on average, and when performing the coupling and QC manually, an average of 4 hours and 23 minutes was required. Accordingly, it was confirmed that when using the apparatus for automated antibody coupling and confirmation thereof of the present invention, the coupling can be performed with uniform accuracy within a short period of time compared to existing methods.

### Example 3

Immuno-diagnosis was performed using the same apparatus for automated antibody coupling and confirmation thereof as that used in Examples 1 and 2 above.

The configuration of the cartridge used at this time (FIG. 11) was as follows.
Cartridge for immuno-diagnosis
Well 1: magnetic particles in MES buffer
Well 2: Antigen in Assay buffer
Well 3: Washing buffer
Well 4: Detection Antibody in Assay buffer
Well 5: SA-PE in Blocking buffer
Well 6: Washing buffer
Well 7: Washing buffer
Well 8: PBS buffer

The magnetic particles obtained by performing automated antibody coupling as in Example 1 were used.

The antibody used was Anti-GFAP and GFAP was used as the antigen.

The configuration of the cartridge used was the same as in FIG. 12.

Repeated three times.

**[Table 3]**

| Antigen concentration (pg/ml) | First time | Second times | Third times | AVG. | STD | CV(%) |
|---|---|---|---|---|---|---|
| 200 | 26716 | 25220 | 25393 | 25776.33 | 668.19 | 2.59 |
| 100 | 14641 | 15430 | 14582 | 14884.33 | 386.60 | 2.60 |
| 50 | 9544 | 9871 | 9553 | 9656.00 | 152.07 | 1.57 |
| 25 | 5753 | 5541 | 5425 | 5573.00 | 135.80 | 2.44 |
| 12.5 | 4910 | 4865 | 5141 | 4972.00 | 120.90 | 2.43 |
| 6.25 | 3868 | 3660 | 3646 | 3724.67 | 101.51 | 2.73 |
| 3.125 | 3086 | 3091 | 3207 | 3128.00 | 55.90 | 1.79 |
| 0 | 1158 | 1134 | 1207 | 1166.33 | 30.38 | 2.60 |

As shown in Table 3, it was confirmed that immuno-diagnosis can be performed using the magnetic particles manufactured in Example 1 of the present invention. In addition, it was confirmed that the variation (CV) in each immuno-diagnosis is also small, which indicates that immuno-diagnosis also has high reproducibility.

In addition, it was confirmed that in the case of the present invention, even at an antigen concentration of 3.125 pg/ml, it can be clearly distinguished from an antigen concentration of 0 pg/ml, and thus it was confirmed that the detection limit of the present invention is at least 3.125 pg/ml.

In the above, although specific parts of the content of the present invention have been described in detail, it will be clear to those skilled in the art that that these specific descriptions are merely preferred embodiments, and the scope of the present invention is not limited thereby. Accordingly, the substantial scope of the present invention will be defined by the appended claims and their equivalents.

## Claims

1. An apparatus for automated antibody coupling and confirmation thereof, comprising:
a cartridge including a plurality of wells;
a magnetic bar installed on the upper part of the wells in the cartridge and moving up and down to enter the inside of the wells; and
an image acquisition unit capable of photographing the inside of one or more of the wells;
wherein as the cartridge, at least one of a cartridge for automated antibody coupling and a cartridge for confirmation after antibody coupling is selectively used, and
at least one well in the cartridge is loaded with magnetic particles.

2. The apparatus for automated antibody coupling and confirmation thereof according to claim 1, wherein the magnetic particle comprises:
a core including a magnetically responsive metal;
a shell layer having a uniform thickness surrounding the core; and
a capture probe introduced onto the shell layer to capture biomaterials,
wherein the magnetic particle is a cut piece from a glass-coated metal microwire.

3. The apparatus for automated antibody coupling and confirmation thereof according to claim 1, wherein the apparatus for automated antibody coupling and confirmation thereof further comprises a tip cover that surrounds the lower part of the magnetic bar and moves up and down simultaneously with or separately from the magnetic bar.

4. The apparatus for automated antibody coupling and confirmation thereof according to claim 1, wherein the cartridge for automated antibody coupling comprises:
a first well loaded with the magnetic particles;
a second well loaded with EDC and NHS;
a third well loaded with capture antibodies;
a fourth well containing a blocking buffer; and
a fifth well containing a washing buffer.

5. The apparatus for automated antibody coupling and confirmation thereof according to claim 4, wherein the first well and the second well further contain a 2-[N-morpholino]ethanesulfonic acid (MES) buffer.

6. The apparatus for automated antibody coupling and confirmation thereof according to claim 4, wherein a buffer well for buffering is further comprised between the fourth and fifth wells, and the third well and the buffer well for buffering contain a 4-(2-hydroxyethyl)-1-piperazineethane sulfonic acid (HEPES) buffer.

7. The apparatus for automated antibody coupling and confirmation thereof according to claim 1, wherein the cartridge for confirmation after antibody coupling comprises:
a first well loaded with magnetic particles to which capture antibodies are coupled;
a second well loaded with second antibodies;
a third well loaded with fluorescent particles; and
a fourth well containing a wash buffer.

8. The apparatus for automated antibody coupling and confirmation thereof according to claim 7, wherein the second antibodies are bound to the capture antibodies through an antigen-antibody reaction, and the fluorescent particles are bound to the second antibodies.

9. The apparatus for automated antibody coupling and confirmation thereof according to claim 7, wherein the first to third wells further contain a blocking buffer.

10. The apparatus for automated antibody coupling and confirmation thereof according to claim 1, wherein the cartridge further comprises a cartridge for immuno-diagnosis.

11. The apparatus for automated antibody coupling and confirmation thereof according to claim 10, wherein the cartridge for immuno-diagnosis comprises:
a first well loaded with magnetic particles to which capture antibodies are coupled;
a second well loaded with antigens;
a third well loaded with detection antibodies;
a fourth well loaded with fluorescent particles; and
a fifth well containing a wash buffer.

12. The apparatus for automated antibody coupling and confirmation thereof according to claim 11, wherein the magnetic particles to which capture antibodies are coupled are a mixture of two or more types of magnetic particles with different lengths, and the magnetic particles with different lengths contain different capture antibodies.

13. The apparatus for automated antibody coupling and confirmation thereof according to claim 1, wherein the apparatus for automated antibody coupling and confirmation thereof performs the coupling process of the magnetic particles and the confirmation process of the coupled magnetic particles by vertically and horizontally moving the magnetic bar.
